# EUROPÄISCHE PATENTSCHRIFT

(11) **EP 3 922 221 B1**
(45) Veröffentlichungstag und Bekanntmachung des Hinweises auf die Patenterteilung: **07.09.2022**
(21) Anmeldenummer: 21176703.3
(22) Anmeldetag: 28.05.2021
(51) Int. Cl.: A61F 5/01

(54) **SPRUNGGELENKORTHESE**
ANKLE PROSTHESIS
ORTHÈSE DE L'ARTICULATION DE LA CHEVILLE

(30) Priorität: 09.06.2020 DE 102020115350
(43) Veröffentlichungstag der Anmeldung: 15.12.2021
(73) Patentinhaber: Albrecht GmbH, 83233 Bernau (DE)
(72) Erfinder: Wolf, Michael, 83059 Kolbermoor (DE)
(74) Vertreter: Flach Bauer Stahl Patentanwälte Partnerschaft mbB

(56) Entgegenhaltungen:
- EP-A1- 3 398 569
- EP-A1- 3 506 858
- WO-A1-2018/187569
- US-A1- 2019 110 917
- US-B2- 8 277 403

## Beschreibung

Die Erfindung betrifft eine Sprunggelenkorthese gemäß dem Oberbegriff des Anspruchs 1.

Sprunggelenkorthesen dienen bekannterweise zur Führung und Stützung des Fußes bei seiner Schwenkbewegung im Bereich des oberen Sprunggelenks, d. h. bei seiner Plantarflexion (Streckung des Fußes nach unten) und Dorsalextension (Abwinkeln des Fußes nach oben). Eine besonders wirkungsvolle Führung und Stützung des Fußes und des Sprunggelenks wird dabei durch Sprunggelenkorthesen erreicht, die starre proximale und distale Schienenelemente aufweisen, die mittels eines Drehgelenks drehbar miteinander verbunden sind.

Aus der US 2019/0110917 A1 ist eine Sprunggelenkorthese gemäß dem Oberbegriff des Anspruchs 1 bekannt, bei der das distale Schienenelement aus einem schwenkbar am proximalen Orthesenteil gelagerten Haltebügel besteht, an dem eine Fußauflage befestigt ist, die zum Aufsetzen des Fußes ausgebildet ist.

Sprunggelenkorthesen dieser Art werden insbesondere auch in Fällen eingesetzt, in denen die Plantarflexion und/oder Dorsalextension des Fußes mittels Federkraft unterstützt werden soll. Hierzu wird die Drehgelenkeinrichtung mit einer Feder ausgestattet, welche das distale Schienenelement und damit den Fuß in die gewünschte Schwenkrichtung drängt.

Bei derartigen Sprunggelenkorthesen ist es von großer Bedeutung, dass die Schwenkbewegung des Fußes im oberen Sprunggelenk nicht behindert wird und die Drehachse der Drehgelenkeinrichtung und die Drehachse des oberen Sprunggelenks bestmöglich fluchten. Hierbei ist zu berücksichtigen, dass sich die Gelenkachse des oberen Sprunggelenks im Verlauf der Bewegung von Plantarflexion nach Dorsalextension verschiebt. Für eine orthetische Versorgung ist es daher erforderlich, eine Kompromissdrehachse (ähnlich wie beim Kniegelenk) zu finden. Diese Kompromissdrehachse kann über die lateralen und medialen Malleoli (Fußknöchel) definiert werden, wobei die Durchstoßpunkte der Drehachse sowohl medial als auch lateral um ein bestimmtes Maß distal und anterior der prominenten Knochenstruktur sowie auf einer Verbindungslinie liegen, welche von medial nach lateral geneigt ist.

Sprunggelenkorthesen, bei denen der die Fußauflage haltende Haltebügel mittels eines Gelenks schwenkbar am proximalen Orthesenteil befestigt ist, sind weiterhin aus EP 3398569 A1, WO 2018/187569 A1 und WO 2019/092119 A1 bekannt.

Bekannte Sprunggelenkorthesen dieser Art lassen sich nicht auf einfache Weise an unterschiedliche Abstände zwischen der Fußsohle und der Drehachse des oberen Sprunggelenks sowie an den Verlauf der Kompromissdrehachse des Sprunggelenks anpassen. Die Schwenkbewegung des Fußes wird daher häufig nicht in optimaler Weise durch die Sprunggelenkorthese abgebildet und durch diese behindert.

Aus der US 8277403 B2 ist weiterhin eine als Knieorthese ausgebildete orthopädische Stützanordnung bekannt, bei der Schienenteile, die am Oberschenkel und am Unterschenkel befestigbar sind, einen Längenverstellmechanismus aufweisen.

Der Erfindung liegt die Aufgabe zugrunde, eine Sprunggelenkorthese zu schaffen, die auf einfache und besonders genaue Weise an die individuelle Anatomie eines Fußes unter besonderer Berücksichtigung des Abstands zwischen Fußsohle und oberem Sprunggelenk anpassbar ist.

Diese Aufgabe wird durch eine Sprunggelenkorthese mit den Merkmalen des Anspruchs 1 gelöst. Vorteilhafte Ausgestaltungen der Erfindung sind in den weiteren Ansprüchen beschrieben.

Die erfindungsgemäße Sprunggelenkorthese umfasst ein proximales Orthesenteil, das eine starre Unterschenkelschiene aufweist, die zur lateralen oder medialen Anordnung an einem Unterschenkel ausgebildet ist. Weiterhin umfasst die Sprunggelenkorthese ein distales Orthesenteil, das einen starren Haltebügel aufweist, an dem eine Fußauflage befestigt ist. Das proximale und das distale Orthesenteil sind mittels einer Drehgelenkeinrichtung drehbar miteinander verbunden, die eine Drehachse bildet. Erfindungsgemäß weist das distale Orthesenteil einen im Bereich der Drehgelenkeinrichtung drehbar gelagerten Schwenkarm auf, an dem der Haltebügel höhenverstellbar derart befestigt ist, dass der Abstand zwischen der Fußauflage und der Drehachse der Drehgelenkeinrichtung veränderbar ist.

Die erfindungsgemäße Sprunggelenkorthese bietet den Vorteil, dass ihre Drehachse auf einfache, schnelle und sehr genaue Weise individuell bei jedem Patienten an die Lage und Drehachse des oberen Sprunggelenks angepasst werden kann. Die Sprunggelenkorthese ist daher für unterschiedlichste Fuß- und Sprunggelenksanatomien geeignet, die insbesondere in Abhängigkeit der Größe der Patienten stark variieren können. Darüber hinaus bietet die erfindungsgemäße Sprunggelenkorthese, wenn sie eine laterale und mediale Orthesenhälfte umfasst, den Vorteil, dass die beiden Orthesenhälften unterschiedlich eingestellt und dadurch auf einfache, schnelle und präzise Weise an den Verlauf einer geneigten Kompromissdrehachse des Sprunggelenks angepasst werden kann.

Vorteilhafterweise weist der Haltebügel einen insbesondere vertikalen Führungsschenkel auf, der auf der lateralen oder medialen Seite des Fußes am Schwenkarm des distalen Orthesenteils verschiebbar und arretierbar befestigt ist.

Vorteilhafterweise weist der Schwenkarm einen Führungskanal auf, in dem der die Fußauflage haltende Haltebügel verschiebbar und arretierbar geführt ist. Ein derartiger Führungskanal kann auf formschlüssige Weise die Kraftübertragung zwischen dem Schwenkarm und dem Haltebügel zuverlässig sicherstellen.

Vorteilhafterweise weist der Schwenkarm eine Schwenkkonsole, die an einem Achselement der Drehgelenkeinrichtung drehbar gelagert ist, und eine an der Schwenkkonsole befestigte Arretiereinrichtung mit mindestens einem verstellbaren Arretierelement zum form- oder kraftschlüssigen Arretieren des Haltebügels am Schwenkarm auf.

Vorteilhafterweise umfasst die Arretiereinrichtung mindestens einen an der Schwenkkonsole verschiebbar gehalterten Rastschieber, der werkzeuglos zwischen einer in Rasteingriff mit dem Haltebügel stehenden Arretierstellung und einer Lösestellung bewegbar ist. Aufgrund der werkzeuglosen Verstellung der Sprunggelenkorthese kann diese auf besonders schnelle und einfache Weise jederzeit bei Bedarf angepasst werden.

Vorteilhafterweise weisen der Rastschieber und der Haltebügel aufeinander abgestimmte Verzahnungen auf, wobei die Verzahnung des Rastschiebers zwischen einer in die Verzahnung des Haltebügels eingreifenden Arretierstellung und einer aus der Verzahnung des Haltebügels entfernten Lösestellung bewegbar ist. Durch eine entsprechend feine Zahnteilung, d.h. durch einen geringen Abstand der Zähne der Verzahnungen, kann die Ausziehlänge des Haltebügels am Schwenkarm entsprechend fein verstellt und arretiert werden. Zweckmäßigerweise weist die Verzahnung des Rastschiebers eine Mehrzahl von Zähnen auf, beispielsweise zwei bis acht Zähne. Es ist jedoch auch denkbar, dass der Rastschieber nur einen einzelnen Zahn oder Stift aufweist, der in Vertiefungen oder Bohrungen des Haltebügels eingreifen kann.

Vorteilhafterweise umfasst die Arretiereinrichtung eine Feder, mit der der Rastschieber in die Raststellung vorgespannt ist. Weiterhin ist es vorteilhaft, wenn der Rastschieber einen Handhabungsabschnitt umfasst, der über die Schwenkkonsole übersteht. Hierdurch kann der Rastschieber auf einfache Weise mit der Hand ergriffen und entgegen der Vorspannkraft der Feder in die Lösestellung gebracht werden, um die Position des Haltebügels relativ zum Schwenkarm zu verändern. Durch Loslassen des Rastschiebers wird dieser durch die Feder dann automatisch wieder in die Arretierstellung zurückbewegt.

Vorteilhafterweise besteht der Rastschieber aus einem vorzugsweise rechteckigen Rahmenelement, das einen Steg der Schwenkkonsole umgibt, wobei die Feder einerseits am Rastschieber und andererseits am Steg abgestützt ist. Der Steg kann hierbei gleichzeitig ein Führungselement für den Rastschieber darstellen.

Vorteilhafterweise sind zwei Rastschieber vorgesehen, die von gegenüberliegenden Seiten her auf den Haltebügel einwirken. Die Kräfte der Rastschieber werden hierdurch gleichmäßig auf beide Seiten des Haltebügels übertragen.

Vorteilhafterweise ist der Haltebügel L-förmig und weist einen horizontalen Befestigungsschenkel zur Befestigung der Fußauflage auf. Der horizontale Befestigungsschenkel ist dabei insbesondere derart ausgebildet, dass eine Fußplatte der Fußauflage auf den horizontalen Befestigungsschenkel aufgelegt und an diesem befestigt werden kann. Hierdurch ergibt sich eine einfache und stabile Befestigungsmöglichkeit für die Fußauflage.

Vorteilhafterweise umfasst die Sprunggelenkorthese eine laterale und mediale Orthesenhälfte, wobei beide Orthesenhälften jeweils das proximale Orthesenteil, das distale Orthesenteil, die Drehgelenkeinrichtung und eine Befestigungswange zur Befestigung der Unterschenkelschiene am Unterschenkel aufweisen, und wobei die laterale Befestigungswange getrennt von der medialen Befestigungswange ausgebildet und mittels flexibler Verbindungselemente mit der medialen Befestigungswange verbunden ist. Die flexiblen Verbindungselemente können insbesondere Klettbändern sein, die an den Befestigungswangen befestigt sind. Durch die Trennung der lateralen und medialen Befestigungswangen kann die Höhenposition des proximalen Orthesenteils zusammen mit der zugeordneten Drehgelenkeinrichtung auf der lateralen oder medialen Seite verändert werden, ohne dass dies durch die Befestigungswange auf der anderen Seite behindert werden würde. Beispielsweise kann die mediale Befestigungswange höher, d.h. mehr proximal, am Unterschenkel als die laterale Befestigungswange angeordnet und in dieser Position mit der lateralen Befestigungswange verbunden werden.

Vorteilhafterweise weist der Haltebügel der lateralen Orthesenhälfte einen ersten, sich von lateral nach medial erstreckenden Befestigungsschenkel zur Befestigung der Fußauflage auf. Der Haltebügel der medialen Orthesenhälfte weist einen zweiten, sich von medial nach lateral erstreckenden Befestigungsschenkel zur Befestigung der Fußauflage auf, wobei der mediale Befestigungsschenkel getrennt vom lateralen Befestigungsschenkel ausgebildet ist. Hierdurch ist es auf einfache Weise möglich, unterschiedlich breite Fußauflagen zwischen der lateralen und medialen Orthesenhälfte anzuordnen. Die Sprunggelenkorthese kann somit nach dem Baukastenprinzip unter Verwendung gleicher Orthesenhälften unterschiedlich breit konfiguriert werden.

Die Erfindung wird nachfolgend anhand der Zeichnungen beispielhaft näher erläutert. Es zeigen:
- Figur 1:: eine Seitenansicht einer erfindungsgemäßen Sprunggelenkorthese, wobei einige Befestigungsmittel im Fuß- und Unterschenkelbereich weggelassen worden sind;
- Figur 2:: eine etwas schematische Ansicht der Sprunggelenkorthese ohne Fußauflage und BeinVerbindungselemente von hinten, wobei die Sprunggelenkorthese an einem linken Fuß befestigt ist, dessen Skelettkonturen schematisch eingezeichnet sind;
- Figur 3:: eine Seitenansicht einer Drehgelenkeinrichtung, eines Schwenkarms und eines Haltebügels ohne Abdeckplatte, wobei lediglich der Haltebügel in leicht räumlicher Darstellung gezeigt ist;
- Figuren 4 und 5:: Seitenansichten der Sprunggelenkorthese in zwei unterschiedlichen Ausziehpositionen des Haltebügels; und
- Figur 6:: eine Seitenansicht der Sprunggelenkorthese entsprechend Figur 5, jedoch mit Abdeckplatte.

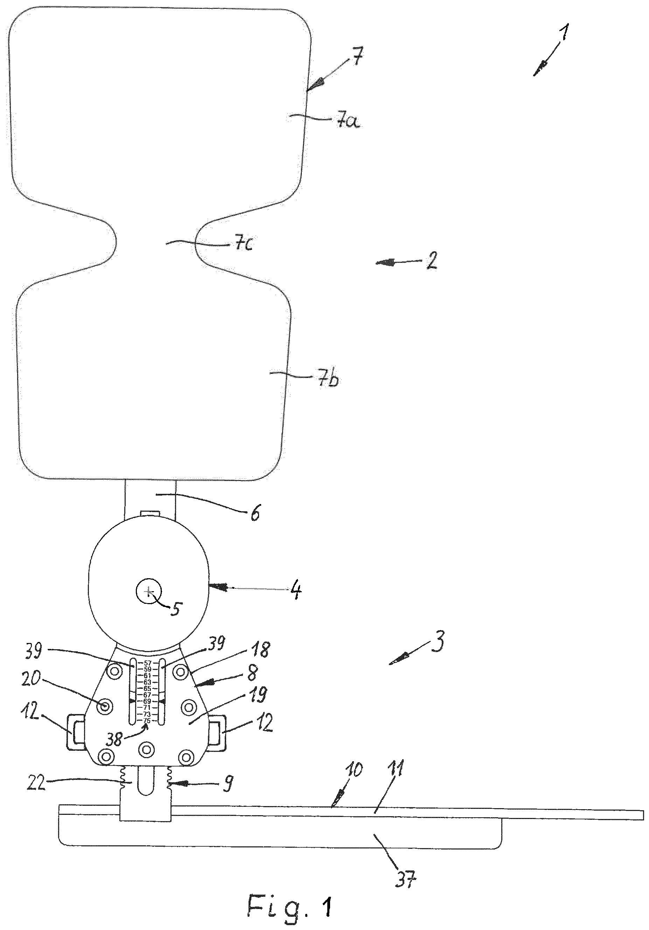

Figur 1 zeigt eine Sprunggelenkorthese 1 mit einem proximalen Orthesenteil 2 und einem distalen Orthesenteil 3, das mittels einer Drehgelenkeinrichtung 4 gelenkig mit dem proximalen Orthesenteil 2 verbunden und um eine Drehachse 5 schwenkbar ist.

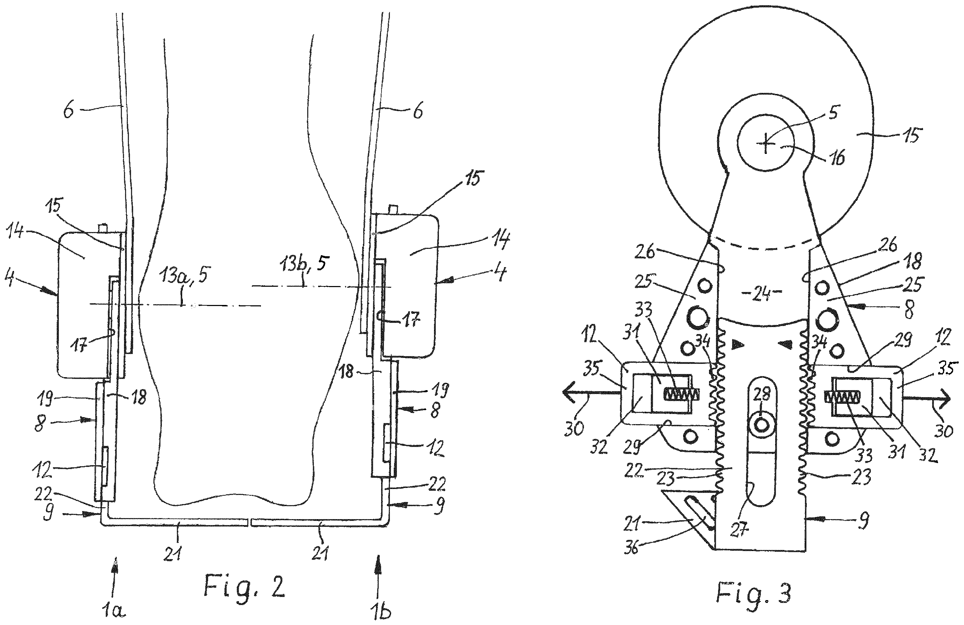

Im dargestellten Ausführungsbeispiel besteht die Sprunggelenkorthese 1, wie aus Figur 2 erkennbar, aus einer lateralen Orthesenhälfte 1a und einer medialen Orthesenhälfte 1b. Beide Orthesenhälften 1a, 1b weisen vorzugsweise den gleichen grundsätzlichen Aufbau auf.

Das proximalen Orthesenteil 2 umfasst eine starre, längliche Unterschenkelschiene 6 und eine vorzugsweise gepolsterte Befestigungswange 7. Die Befestigungswange 7 ist derart ausgebildet, dass sie sich seitlich an den Unterschenkel anlegen lässt, wobei sie den Unterschenkel in dessen Umfangsrichtung überwiegend halbkreisförmig umschließt. Vorzugsweise besteht die Befestigungswange 7 aus einem flexiblen Material, das sich der Kontur des Unterschenkels leicht anpasst. Die Befestigungswange 7 wird mittels nicht dargestellter flexibler Verbindungselemente, insbesondere in der Form von Gurten mit Klettverschluss, am Unterschenkel befestigt, wobei die Befestigungswangen 7 der beiden Orthesenhälften 1a, 1b zusammengegurtet werden.

Die Unterschenkelschiene 6 erstreckt sich von der Drehgelenkeinrichtung 4 proximal über die überwiegende Länge der Befestigungswange 7 und ist fest mit dieser verbunden. Beispielsweise kann sich die Unterschenkelschiene 6 in eine längliche Aufnahmetasche der Befestigungswange 7 hineinerstrecken, die sich mittig und über den wesentlichen Teil der Länge des Befestigungswange 7, d.h. in Figur 1 vertikal, erstreckt.

Die Sprunggelenkorthese 1 ist derart ausgebildet, dass sich die Unterschenkelschiene 6 der lateralen Orthesenhälfte 1a auf der lateralen Seite längs des Unterschenkels erstreckt, während sich die Unterschenkelschiene 6 der medialen Orthesenhälfte 1b auf der medialen Seite längs des Unterschenkels erstreckt.

Wie aus Figur 1 weiter ersichtlich, umfasst die Befestigungswange 7 einen proximalen Abschnitt 7a und einen distalen Abschnitt 7b, wobei beide Abschnitte 7a, 7b durch einen wesentlich schmäleren Verbindungssteg 7c miteinander verbunden sind. Hierdurch lassen sich die beiden Abschnitte 7a und 7b unabhängig voneinander und unter Vermeidung von Faltenbildung an den Unterschenkel anlegen.

Wie aus Figur 1 weiter ersichtlich ist, umfasst das distale Orthesenteil 3 einen um die Drehachse 5 drehbaren, starren Schwenkarm 8 und einen ebenfalls starren Haltebügel 9. Der Haltebügel 9 ist höhenverstellbar am Schwenkarm 8 befestigt.

Am distalen Ende des Haltebügels 9 ist eine Fußauflage 10 befestigt. Diese Fußauflage 10 umfasst eine stabile Fußplatte 11, die derart ausgebildet ist, dass der Fuß eines Patienten auf die Fußplatte 11 oder auf eine nicht dargestellte, auf der Fußplatte befestigte zusätzliche Auflage aufgesetzt werden kann. Die Fußauflage 10 umfasst weiterhin eine Laufsohle 37, die an der Unterseite der Fußplatte 11 befestigt ist.

Figur 1 zeigt ferner zwei Rastschieber 12, die am Schwenkarm 8 verschiebbar geführt sind und zum Arretieren des Haltebügels 9 in einer bestimmten Höhenstellung relativ zum Schwenkarm 8 dienen. Die Rastschieber 12 wirken von gegenüberliegenden Seiten her auf den Haltebügel 9 ein und können manuell auseinandergezogen werden, wodurch die Arretierung aufgehoben wird und der Haltebügel 9 relativ zum Schwenkarm 8 in dessen Längsrichtung verschoben werden kann. Hierdurch kann der Abstand zwischen der Fußplatte 11 und der Drehgelenkeinrichtung 4 bzw. Drehachse 5 verändert werden.

Figur 2 zeigt die an einem linken Fuß angelegte Sprunggelenkorthese 1 in etwas schematischer Darstellung, wobei die Verbindungselemente zur Befestigung der Sprunggelenkorthese 1 am Unterschenkel sowie die Fußauflage 10 weggelassen sind. In Figur 2 ist weiterhin die Skelettkontur im Bereich und in Nachbarschaft des oberen Sprunggelenks eingezeichnet. Wie ersichtlich, liegt die durch den lateralen Knöchel (Malleolus) gebildete Drehachse 13a etwas tiefer, d.h. mehr distal, als die durch den medialen Knöchel gebildete Drehachse 13b. Die Drehgelenkeinrichtungen 4 der beiden Orthesenhälften 1a, 1b sind derart positioniert, dass ihre Drehachsen 5 mit den benachbarten Malleoli-Drehachsen 13a, 13b zusammenfallen. Die Drehgelenkeinrichtungen 4 der beiden Orthesenhälften 1a, 1b haben somit unterschiedliche Abstände zum distalen Ende der Haltebügel 9.

Die Drehgelenkeinrichtung 4 der Orthesenhälften 1a, 1b umfasst, wie aus Figur 2 weiter ersichtlich, ein napfförmiges Gehäuse 14, das auf seiner dem Fuß zugewandten Seite mit einer Platte 15 abgedeckt und fest mit dieser verbunden ist. Gehäuse 14 und Platte 15 sind mit dem distalen Ende der Unterschenkelschiene 6 fest verbunden. Im Gehäuse 14 befindet sich ein in Figur 1 schematisch dargestelltes Achselement 16, durch das die Drehachse 5 gebildet wird. Der Schwenkarm 8 ist am Achselement 16 schwenkbar gelagert. Eine in der Umfangswand des Gehäuses 14 vorgesehene Aussparung 17 schafft den notwendigen Freiraum für die Schwenkbewegung für den in das Gehäuse 14 hineinragenden Schwenkarm 8.

Die Drehgelenkeinrichtung 4 weist vorzugsweise eine nicht dargestellte Feder auf, durch welche das distale Orthesenteil 3 in eine Schwenkrichtung, d.h. in Richtung Dorsalextension oder Plantarflexion, vorgespannt ist. Die Vorspannungseinrichtung kann insbesondere derart ausgebildet sein, dass die Fußauflage 10 von einer Stellung, in welcher der vordere Bereich (Zehenbereich) nach unten geneigt ist, nach oben in die in Figur 1 gezeigte horizontale Stellung oder darüber hinaus gedrängt wird. Dies unterstützt das Anheben des Fußes, wenn Patienten dies nicht ohne Unterstützung bewerkstelligen können. Darüber hinaus können im Gehäuse 14 auch weitere Einrichtungen angeordnet sein, beispielsweise Einrichtungen zum Abkoppeln der Federkraft, zur Einstellung der Stärke der Federkraft oder zur Begrenzung des Schwenkwinkels, über den das distale Orthesenteil 3 relativ zum proximalen Orthesenteil 2 schwenkbar ist.

Aus Figur 2 ist weiterhin ersichtlich, dass der Schwenkarm 8 eine Schwenkkonsole 18 und eine Abdeckplatte 19 umfasst. Die Schwenkkonsole 18 erstreckt sich mit einem proximalen Abschnitt in das Gehäuse 14 hinein und ist am Achselement 16 drehbar gelagert. Der distal über das Gehäuse 14 hinausragende Abschnitt der Schwenkkonsole 18 ist zumindest überwiegend mittels der Abdeckplatte 19 abgedeckt, die mittels Schrauben 20 (Figur 1) an der Schwenkkonsole 18 festgeschraubt ist.

Figur 3 zeigt eine etwas schematische Seitenansicht der Platte 15 der Drehgelenkeinrichtung 4 sowie des distalen Orthesenteils 3, wobei lediglich das untere Ende des Haltebügels 9 räumlich dargestellt ist, um dessen Form zu verdeutlichen.

Der Haltebügel 9 ist L-förmig ausgebildet und weist einen horizontalen Befestigungsschenkel 21, der die Fußplatte 11 vorzugsweise untergreift und mit dieser fest verbunden ist, und einen vertikalen Führungsschenkel 22 auf, mit dem der Haltebügel 9 am Schwenkarm 8 in dessen Längsrichtung verschiebbar geführt ist. An beiden Seiten des Führungsschenkels 22 ist eine Verzahnung 23 vorgesehen, die sich im dargestellten Ausführungsbeispiel über den überwiegenden Teil der Länge des Führungsschenkels 22 erstreckt.

Die Schwenkkonsole 18 weist einen Führungskanal 24 auf, der sich mittig längs der Schwenkkonsole 18 erstreckt. Im gezeigten Ausführungsbeispiel besteht der Führungskanal 24 aus einer nut- oder rinnenartigen Vertiefung, die durch zwei seitlich erhöhte bzw. dickere Bereiche 25 der Schwenkkonsole 18 begrenzt wird. Hierdurch werden zwei parallele seitliche Führungsflächen 26 gebildet. Die Tiefe des Führungskanals 24 ist nur geringfügig größer als die Dicke des Führungsschenkels 22 des Haltebügels 9, so dass der Haltebügel 9 auch quer zur Hauptebene der Schwenkkonsole 18 geführt ist, wenn die Abdeckplatte 19 auf die seitlich erhöhten Bereiche 25 aufgesetzt und mit diesen verschraubt ist.

Der Führungsschenkel 22 des Haltebügels 9 weist ferner einen Längsschlitz 27 auf, in den ein Führungszapfen 28 der Schwenkkonsole 18 hineinragt. Durch diesen Führungszapfen 28 ist der Haltebügel 9 sowohl bei einem Verschieben längs des Führungskanals 24 als auch im arretierten Zustand zusätzlich an der Schwenkkonsole 18 geführt. Weiterhin kann der Führungszapfen 28 als Endanschlag dienen, mit dem der Ausziehweg und/oder Einschubweg des Haltebügels 9 aus dem bzw. in den Schwenkarm 8 begrenzt wird.

Die beiden Rastschieber 12 sind rahmenförmig ausgebildet und haben vorzugsweise, wie aus dem dargestellten Ausführungsbeispiel ersichtlich, eine rechteckige Form. Die Rastschieber 12 sind in Nuten 29, die sich in den seitlich erhöhten Bereichen 25 der Schwenkkonsole 18 befinden, quer zur Längsrichtung des Führungskanals 24 verschiebbar, wie durch die Pfeile 30 angedeutet. Ein gegenüber den Nuten 29 erhabener Bereich oder Steg 31 greift in den inneren freien Raum 32 der Rastschieber 12 ein und kann ein zusätzliches Führungselement für die Rastschieber 12 bilden.

Die Stege 31 dienen weiterhin als Anschlagfläche für eine Feder 33, die im gezeigten Ausführungsbeispiel als Druckfeder ausgebildet ist. Das andere Ende der Federn 33 ist an den Rastschiebern 12 abgestützt. Aufgrund dieser Anordnung üben die Federn 33 eine permanente Vorspannkraft auf die Rastschieber 12 aus, welche diese in Richtung des Führungsschenkels 22 des Haltebügels 9 drängt.

Weiterhin weisen die Rastschieber 12 jeweils eine Verzahnung 34 auf, welche an die Verzahnung 23 des Führungsschenkels 22 angepasst ist und in diese formschlüssig eingreifen kann. Die Federn 33 drängen diese Verzahnung 34 in diese Eingriffsstellung, wodurch eine Verschiebung des Haltebügels 9 blockiert wird. Werden die Rastschieber 12 auseinandergezogen, wie durch die Pfeile 30 veranschaulicht, gelangen die Verzahnungen 34 der Rastschieber 12 außer Eingriff mit der Verzahnung 23 des Haltebügels 9, so dass dieser in Längsrichtung des Führungskanals 24 verschoben werden kann.

Die Rastschieber 12 stehen jeweils seitlich über die Schwenkkonsole 18 vor. Der vorstehende Teil bildet einen Handhabungsabschnitt 35, der auf einfache Weise mit zwei Fingern gegriffen werden kann, um die Rastschieber 12 auseinander zu ziehen.

Wie aus Figur 2 ersichtlich, weist der Haltebügel 9 der lateralen Orthesenhälfte 1a einen ersten, sich von lateral nach medial erstreckenden Befestigungsschenkel 21 zur Befestigung der Fußauflage 10 auf. Weiterhin weist der Haltebügel 9 der medialen Orthesenhälfte 1b einen zweiten, sich von medial nach lateral erstreckenden Befestigungsschenkel 21 zur Befestigung der Fußauflage 10 auf. Der laterale Befestigungsschenkel 1a ist hierbei getrennt vom medialen Befestigungsschenkel 1b ausgebildet. Auf diese Weise kann der seitliche Abstand der beiden Orthesenhälften 1a, 1b an die Breite des Fußes im Bereich des oberen Sprunggelenks und/oder an unterschiedlich breite Fußauflagen 10 angepasst werden.

Die Befestigung der Fußauflage 10 an den Befestigungsschenkeln 21 folgt vorzugsweise durch nicht dargestellte Schrauben, die durch Langlöcher 36 (Figur 3) des Befestigungsschenkels 21 hindurchgeführt und mit der Fußplatte 11 verschraubt werden. Die Langlöcher 36 ermöglichen die variable Positionierung der Haltebügel 9 relativ zur Fußauflage 10 in seitlicher Richtung.

Die Befestigungsschenkel 21 der lateralen Orthesenhälfte 1a und der medialen Orthesenhälfte 1b, die vorzugsweise in derselben Ebene liegen, sind zwar voneinander getrennt ausgebildet, können sich jedoch mittig etwas überlappen. Zu diesem Zweck können die Befestigungsschenkel 21 freie Endbereiche aufweisen, die dorsal bzw. ventral versetzt zueinander angeordnet sind.

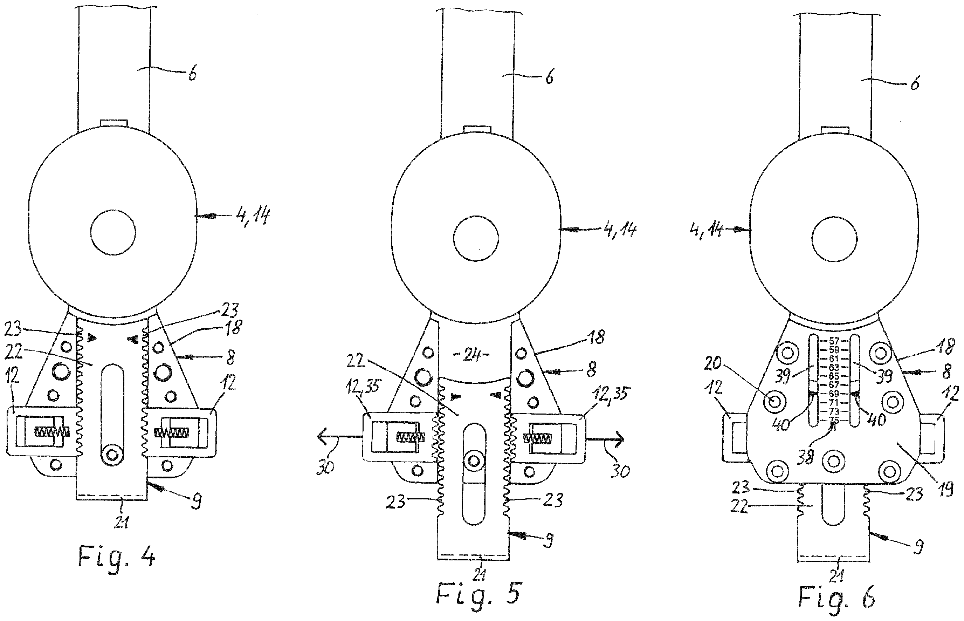

Anhand der Figuren 4 bis 6 wird im Folgenden die Verstellung des Haltebügels 9 kurz beschrieben.

In Figur 4 ist der Haltebügel 9 in seiner maximal proximalen Stellung durch die Rastschieber 12 arretiert. Der Abstand zwischen den Befestigungsschenkeln 21 und damit der Fußauflage 10 und der Drehgelenkeinrichtung 4 bzw. Drehachse 5 ist minimal. Die Rastschieber 12 werden von den Federn 33 in ihrer Arretierstellung gehalten.

Figur 5 zeigt eine Abbildung, in der die beiden Rastschieber 12 manuell in Richtung der Pfeile 30 auseinandergezogen worden sind, so dass die Verzahnungen 34 der Rastschieber 12 außer Eingriff mit den Verzahnungen 23 des Haltebügels 9 sind. Der Haltebügel 9 kann nach unten, d.h. in distaler Richtung, derart verschoben werden, dass sich der Abstand zwischen den Befestigungsschenkeln 21 und der Drehachse 5 vergrößert. Nach dem Loslassen der Rastschieber 12 werden diese durch die Federn 33 wieder in die Arretierstellung zurückbewegt.

Figur 6 zeigt dieselbe Position des Haltebügels 9 wie in Figur 5, jedoch mit aufgesetzter Abdeckplatte 19. Wie ersichtlich, kann die Abdeckplatte 19 eine Skala 38 und Sichtfenster 39 aufweisen, durch die Markierungen 40 der Haltebügel 9 hindurch sichtbar sind, welche die Ausziehposition der Haltebügels 9 relativ zum Schwenkarm 8 angeben. Die Skala 38 kann beispielsweise Millimeterangaben umfassen, welche den Abstand zwischen der Fußauflage 10 und der Drehachse 5 angeben.

Das in den Zeichnungen dargestellte Ausführungsbeispiel der Sprunggelenkorthese 1 kann in vielfältiger Weise variiert werden. Anstelle einer Sprunggelenkorthese 1 mit einer lateralen Orthesenhälfte 1a und einer medialen Orthesenhälfte 1b ist es auch denkbar, die Erfindung bei Sprunggelenkorthesen anzuwenden, die nur auf einer Seite des Fußes angeordnet werden. Anstelle von zwei Rastschiebern 12 kann die Arretiereinrichtung auch anders gestaltet sein, beispielsweise in Form eines oder mehrerer Arretierstifte, die mit dem Haltebügel 9 in und außer Verriegelungseingriff treten können.

## Patentansprüche

1. Sprunggelenkorthese mit
- einem proximalen Orthesenteil (2), das eine starre Unterschenkelschiene (6) aufweist, die zur lateralen oder medialen Anordnung an einem Unterschenkel ausgebildet ist,
- einem distalen Orthesenteil (3), das einen starren Haltebügel (9) aufweist, an dem eine Fußauflage (10) befestigt ist,
- eine Drehgelenkeinrichtung (4), die das proximale Orthesenteil (2) und das distale Orthesenteil (3) drehbar miteinander verbindet und eine Drehachse (5) bildet,
**dadurch gekennzeichnet, dass** das distale Orthesenteil (3) einen im Bereich der Drehgelenkeinrichtung (4) drehbar gelagerten Schwenkarm (8) aufweist, an dem der Haltebügel (9) höhenverstellbar befestigt ist, derart, dass der Abstand zwischen der Fußauflage (10) und der Drehachse (5) der Drehgelenkeinrichtung (4) veränderbar ist.

2. Sprunggelenkorthese nach Anspruch 1, **dadurch gekennzeichnet, dass** der Haltebügel (9) einen Führungsschenkel (22) aufweist, der auf der lateralen oder medialen Seite des Fußes am Schwenkarm (8) des distalen Orthesenteils (2) verschiebbar und arretierbar befestigt ist.

3. Sprunggelenkorthese nach einem der vorhergehenden Ansprüche, **dadurch gekennzeichnet, dass** der Schwenkarm (8) einen Führungskanal (24) aufweist, in dem der die Fußauflage (10) haltende Haltebügel (9) verschiebbar und arretierbar geführt ist.

4. Sprunggelenkorthese nach einem der vorhergehenden Ansprüche, **dadurch gekennzeichnet, dass** der Schwenkarm (8) eine Schwenkkonsole (18), die an einem Achselement (16) der Drehgelenkeinrichtung (4) drehbar gelagert ist, und eine an der Schwenkkonsole (18) befestigte Arretiereinrichtung mit mindestens einem verstellbaren Arretierelement zum form- oder kraftschlüssigen Arretieren des Haltebügels (9) am Schwenkarm (8) aufweist.

5. Sprunggelenkorthese nach Anspruch 4, **dadurch gekennzeichnet, dass** die Arretiereinrichtung mindestens einen an der Schwenkkonsole (18) verschiebbar gehalterten Rastschieber (12) umfasst, der werkzeuglos zwischen einer in Rasteingriff mit dem Haltebügel (9) stehenden Arretierstellung und einer Lösestellung bewegbar ist.

6. Sprunggelenkorthese nach Anspruch 5, **dadurch gekennzeichnet, dass** der Rastschieber (12) und der Haltebügel (9) aufeinander abgestimmte Verzahnungen (34, 23) aufweisen, wobei die Verzahnung (34) des Rastschiebers (12) zwischen einer in die Verzahnung (23) des Haltebügels (9) eingreifenden Arretierstellung und einer aus der Verzahnung (23) des Haltebügels (9) entfernten Lösestellung bewegbar ist.

7. Sprunggelenkorthese nach Anspruch 5 oder 6, **dadurch gekennzeichnet, dass** die Arretiereinrichtung eine Feder (33) umfasst, mit der der Rastschieber (12) in die Raststellung vorgespannt ist.

8. Sprunggelenkorthese nach einem der Ansprüche 5 bis 7, **dadurch gekennzeichnet, dass** der Rastschieber (12) einen Handhabungsabschnitt (35) umfasst, der über die Schwenckonsole (18) übersteht.

9. Sprunggelenkorthese nach Anspruch 7 oder 8, **dadurch gekennzeichnet, dass** der Rastschieber (12) aus einem Rahmenelement besteht, das einen Steg (31) der Schwenckonsole (18) umgibt, wobei die Feder (33) einerseits am Rastschieber (12) und andererseits am Steg (31) abgestützt ist.

10. Sprunggelenkorthese nach einem der Ansprüche 5 bis 9, **dadurch gekennzeichnet, dass** zwei Rastschieber (12) vorgesehen sind, die von gegenüberliegenden Seiten her auf den Haltebügel (9) einwirken.

11. Sprunggelenkorthese nach einem der vorhergehenden Ansprüche, **dadurch gekennzeichnet, dass** der Haltebügel (9) L-förmig ist und einen horizontalen Befestigungsschenkel (21) zur Befestigung der Fußauflage (10) aufweist.

12. Sprunggelenkorthese nach einem der vorhergehenden Ansprüche, **dadurch gekennzeichnet, dass** die Sprunggelenkorthese (1) eine laterale und eine mediale Orthesenhälfte (1a, 1b) umfasst, wobei beide Orthesenhälften (1a, 1b) jeweils das proximale Orthesenteil (2), das distale Orthesenteil (3), die Drehgelenkeinrichtung (4) und eine Befestigungswange (7) zur Befestigung der Unterschenkelschiene (6) am Unterschenkel aufweisen, und wobei die laterale Befestigungswange getrennt von der medialen Befestigungswange ausgebildet und mittels flexibler Verbindungselemente mit der medialen Befestigungswange verbunden ist.

13. Sprunggelenkorthese nach Anspruch 12, **dadurch gekennzeichnet, dass** der Haltebügel (9) der lateralen Orthesenhälfte (1a) einen ersten, sich von lateral nach medial erstreckenden Befestigungsschenkel (21) zur Befestigung der Fußauflage (10) aufweist, und dass der Haltebügel (9) der medialen Orthesenhälfte (1b) einen zweiten, sich von medial nach lateral erstreckenden Befestigungsschenkel (21) zur Befestigung der Fußauflage (10) aufweist, wobei der laterale Befestigungsschenkel getrennt vom medialen Befestigungsschenkel ausgebildet ist.

14. Sprunggelenkorthese nach einem der vorhergehenden Ansprüche, **dadurch gekennzeichnet, dass** die Fußauflage (10) eine Fußplatte (11) aufweist, die zum Aufsetzen des Fußes ausgebildet ist, wobei der Befestigungsschenkel (21) des Haltebügels (9) die Fußplatte (11) untergreift.

## Claims

1. Ankle joint orthosis having
- a proximal orthosis part (2) having a rigid lower leg splint (6) designed to be arranged laterally or medially on a lower leg,
- a distal orthosis part (3) having a rigid retainer (9) to which a foot support (10) is attached,
- a swivel joint device (4) which rotatably connects the proximal orthosis part (2) and the distal orthosis part (3) to each other and forms an axis of rotation (5),
**characterised in that** the distal orthosis part (3) has a swivel arm (8) which is rotatably mounted in the region of the swivel joint device (4) and to which the retainer (9) is attached in a height-adjustable manner in such a way that the distance between the foot support (10) and the axis of rotation (5) of the swivel joint device (4) can be changed.

2. Ankle joint orthosis according to claim 1, **characterised in that** the retainer (9) has a guide leg (22) which is movably and lockably attached to the swivel arm (8) of the distal orthosis part (2) on the lateral or medial side of the foot.

3. Ankle joint orthosis according to one of the preceding claims, **characterised in that** the swivel arm (8) has a guide channel (24) in which the retainer (9) holding the foot support (10) is guided in a moveable and lockable manner.

4. Ankle joint orthosis according to one of the preceding claims, **characterised in that** the swivel arm (8) has a swivel bracket (18), which is rotatably mounted on an axle element (16) of the swivel joint device (4), and a locking device, which is attached to the swivel bracket (18), with at least one adjustable locking element for the positive or non-positive locking of the retainer (9) on the swivel arm (8).

5. Ankle joint orthosis according to claim 4, **characterised in that** the locking device comprises at least one latching slide (12) which is movably mounted on the swivel bracket (18) and which is moveable without tools between a locking position in latching engagement with the retainer (9) and a release position.

6. Ankle joint orthosis according to claim 5, **characterised in that** the latching slide (12) and the retainer (9) have teeth (34, 23) which are geared to one another, wherein the teeth (34) of the latching slide (12) can be moved between a locking position engaging in the teeth (23) of the retainer (9) and a release position at a distance from the teeth (23) of the retainer (9).

7. Ankle joint orthosis according to claim 5 or 6, **characterised in that** the locking device comprises a spring (33) with which the latching slide (12) is pretensioned into the locking position.

8. Ankle joint orthosis according to one of claims 5 to 7, **characterised in that** the latching slide (12) comprises a handle portion (35) which projects beyond the swivel bracket (18).

9. Ankle joint orthosis according to claim 7 or 8, **characterised in that** the latching slide (12) consists of a frame element which surrounds a crosspiece (31) of the swivel bracket (18), wherein the spring (33) is supported on the one hand on the latching slide (12) and on the other hand on the crosspiece (31).

10. Ankle joint orthosis according to one of the claims 5 to 9, **characterised in that** two latching slides (12) are provided which act on the retainer (9) from opposite sides.

11. Ankle joint orthosis according to one of the preceding claims, **characterised in that** the retainer (9) is L-shaped and has a horizontal attachment leg (21) for attaching the foot support (10).

12. Ankle joint orthosis according to one of the preceding claims, **characterised in that** the ankle joint orthosis (1) comprises a lateral and a medial orthosis half (1a, 1b), wherein both orthosis halves (1a, 1b) each have the proximal orthosis part (2), the distal orthosis part (3), the swivel joint device (4) and an attachment flange (7) for attaching the lower leg splint (6) to the lower leg, and wherein the lateral attachment flange is formed separately from the medial attachment flange and is connected to the medial attachment flange by means of flexible connecting elements.

13. Ankle joint orthosis according to claim 12, **characterised in that** the retainer (9) of the lateral orthosis half (1a) has a first attachment leg (21), which extends from lateral to medial, for attaching the foot support (10), and the retainer (9) of the medial orthosis half (1b) has a second attachment leg (21), which extends from medial to lateral, for attaching the foot support (10), wherein the lateral attachment leg is formed separately from the medial attachment leg.

14. Ankle joint orthosis according to one of the preceding claims, **characterised in that** the foot support (10) has a foot plate (11) which is designed for the foot to be placed on, wherein the attachment leg (21) of the retainer (9) engages under the foot plate (11).

## Revendications

1. Orthèse d'articulation de cheville pourvue
- d'une partie d'orthèse proximale (2) qui présente un rail pour le mollet (6) rigide qui est conçu pour un agencement latéral ou médian sur un mollet,
- d'une partie d'orthèse distale (3) qui présente un support de maintien (9) rigide sur lequel est fixé un repose-pied (10),
- un dispositif d'articulation pivotante (4) qui relie la partie d'orthèse proximale (2) et la partie d'orthèse distale (3) l'une à l'autre en pouvant pivoter et forme un axe de rotation (5),
**caractérisée en ce que** la partie d'orthèse distale (3) présente un bras pivotant (8), logé en pouvant être mis en rotation dans la zone du dispositif d'articulation pivotante (4), sur lequel le support de maintien (9) est fixé en étant réglable en hauteur, de telle manière que la distance entre le repose-pied (10) et l'axe de rotation (5) du dispositif d'articulation pivotante (4) puisse être modifiée.

2. Orthèse d'articulation de cheville selon la revendication 1, **caractérisée en ce que** le support de maintien (9) présente un épaulement de guidage (22) qui peut être déplacé ou être fixé, verrouillé sur le côté latéral ou médian du pied sur le bras pivotant (8) de la partie d'orthèse distale (2).

3. Orthèse d'articulation de cheville selon l'une des revendications précédentes, **caractérisée en ce que** le bras pivotant (8) présente un canal de guidage (24) dans lequel le support de maintien (9) supportant le repose-pied (10) peut être mené en étant coulissable et verrouillable.

4. Orthèse d'articulation de cheville selon l'une des revendications précédentes, **caractérisée en ce que** le bras pivotant (8) présente une console de pivotement (18) qui est logée en pouvant être mise en rotation sur un élément d'axe (16) du dispositif d'articulation pivotante (4), et un dispositif de verrouillage fixé sur la console de pivotement (18) avec au moins un élément de verrouillage réglable pour un verrouillage par complémentarité des formes ou des matières du support de maintien (9) sur le bras pivotant (8).

5. Orthèse d'articulation de cheville selon la revendication 4, **caractérisée en ce que** le dispositif de verrouillage comprend au moins un coulisseau de verrouillage (12), maintenu coulissable sur la console de pivotement (18), qui peut être déplacé sans outil entre une position de verrouillage placée en prise de verrouillage avec le support de maintien (9) et une position libérée.

6. Orthèse d'articulation de cheville selon la revendication 5, **caractérisée en ce que** le coulisseau de verrouillage (12) et le support de maintien (9) présentent des indentations (34, 23) se complétant, où l'indentation (34) du coulisseau de verrouillage (12) peut se déplacer entre une position de verrouillage mettant en prise l'indentation (23) du support de maintien (9) et une position libérée éloignant l'indentation (23) du support de maintien (9).

7. Orthèse d'articulation de cheville selon la revendication 5 ou la revendication 6, **caractérisée en ce que** le dispositif de verrouillage comprend un ressort (33) avec lequel le coulisseau de verrouillage (12) est précontraint dans la position de verrouillage.

8. Orthèse d'articulation de cheville selon l'une des revendications précédentes, **caractérisée en ce que** le coulisseau de verrouillage (12) comprend un segment de maniabilité (35) qui dépasse au-dessus de la console de pivotement (18).

9. Orthèse d'articulation de cheville selon la revendication 7 ou la revendication 8, **caractérisée en ce que** le coulisseau de verrouillage (12) est constitué d'un élément d'encadrement qui entoure une tige (31) de la console de pivotement (18), où le ressort (33) est appuyé d'une part sur le coulisseau de verrouillage (12) et d'autre part sur la tige (31).

10. Orthèse d'articulation de cheville selon l'une des revendications 5 à 9, **caractérisée en ce que** deux coulisseaux de verrouillage (12) sont prévus qui agissent à partir des côtés de part et d'autre sur le support de maintien (9).

11. Orthèse d'articulation de cheville selon l'une des revendications précédentes, **caractérisée en ce que** le support de maintien (9) est en forme de L et présente un épaulement de fixation (21) horizontal pour la fixation du repose-pied (10).

12. Orthèse d'articulation de cheville selon l'une des revendications précédentes, **caractérisée en ce que** l'orthèse d'articulation de cheville (1) comprend des moitiés d'orthèse (1a, 1b) latérale et médiane, les deux moitiés d'orthèse (1a, 1b) présentant respectivement la partie d'orthèse proximale (2), la partie d'orthèse distale (3), le dispositif d'articulation pivotante (4) et une joue de fixation (7) pour la fixation du rail de mollet (6) sur le mollet, et où la joue de fixation latérale est conçue séparée de la joue de fixation médiane et est reliée avec la joue de fixation médiane au moyen d'éléments de liaison souples.

13. Orthèse d'articulation de cheville selon la revendication 12, **caractérisée en ce que** le support de maintien (9) de la moitié d'orthèse latérale (1a) présente un premier épaulement de fixation (21) s'étendant latéralement pour la fixation du repose-pied (10), et que le support de maintien (9) de la moitié d'orthèse médiane (1b) présente un deuxième épaulement de fixation (21) s'étendant de la position médiane vers la position latérale pour la fixation du repose-pied (10), où l'épaulement de fixation latéral est conçu séparé de l'épaulement de fixation médian.

14. Orthèse d'articulation de cheville selon l'une des revendications précédentes, **caractérisée en ce que** le repose-pied (10) présente une plaque pour le pied (11) qui est conçue pour poser le pied, où l'épaulement de fixation (21) du support de maintien (9) se met en-dessous de la plaque pour le pied (11).
